# EUROPEAN PATENT APPLICATION

(11) **EP 4 559 518 A1**
(43) Date of publication of application: **28.05.2025**
(21) Application number: 24213881.6
(22) Date of filing: 19.11.2024
(51) Int. Cl.: A61N 5/10

(54) **RADIATION TREATMENT PLAN OPTIMIZATION METHOD AND APPARATUS**

(30) Priority: 22.11.2023 US 202318517079
(71) Applicant: Siemens Healthineers International AG, 6312 Steinhausen (CH)
(72) Inventor: KUUSELA, Esa, 02320 Espoo (FI); KAUPPINEN, Juha, 02770 Espoo (FI); PELTOLA, Jarkko, 04300 Tuusula (FI); SABEL, Martin, 6332 Hagendorn (CH); BOYLAN, Christopher, 00500 Helsinki (FI)
(74) Representative: Mathisen & Macara LLP

(57) **Abstract**

A control circuit accesses 201 patient information for a patient and optimizes 203 a radiation treatment plan for that patient as a function of both the patient information and a dose distribution objective function, wherein a corresponding available solution space is limited as a function of at least one three-dimensional conformal solution.

## Description

### Technical Field

These teachings relate generally to optimizing a radiation treatment plan that is usable for treating a patient's planning target volume with energy pursuant to the treatment plan and more particularly to optimizing an energy-based treatment plan.

### Background

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Existing techniques in the foregoing regards do not, however, fully meet all needs of all application settings.

### Summary

In one aspect, the present invention provides a method as defined in claim 1. Optional features are specified in the dependent claims.

In another aspect, the present invention provides apparatus as defined in claim 10. Optional features are specified in the dependent claims.

### Brief Description of the Drawings

The above needs are at least partially met through provision of the radiation treatment plan optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic representation as configured in accordance with various embodiments of these teachings;
FIG. 4 comprises a schematic representation as configured in accordance with various embodiments of these teachings; and
FIG. 5 comprises a schematic representation as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### Detailed Description

Generally speaking, pursuant to these various embodiments, a control circuit accesses patient information for a patient and optimizes a radiation treatment plan for that patient as a function of both the patient information and a dose distribution objective function, wherein a corresponding available solution space is limited as a function of at least one three-dimensional conformal solution. (Generally speaking, a solution space refers to the set of all possible points of an optimization problem that satisfy the problem's constraints and hence comprises an initial set of candidate solutions to the problem.)

By one approach, the corresponding available solution space can be limited as a function of at least one three-dimensional conformal solution by, at least in part, limiting the available solution space to a three-dimensional conformal solution. By one approach, the corresponding available solution space can be limited as a function of at least one three-dimensional conformal solution by, at least in part, limiting the available solution space to three-dimensional conformal partial solutions. By yet another approach, the corresponding available solution space can be limited as a function of at least one three-dimensional conformal solution by, at least in part, limiting the available solution space to conformal multi-leaf collimator solutions.

By one approach, optimizing the radiation treatment plan as a function of the dose distribution objective function can comprise, at least in part, optimizing the radiation treatment plan as a function of inverse treatment planning.

By one approach, the aforementioned patient information can include information regarding a plurality of treatment targets in the patient. In such a case, the radiation treatment plan can comprise a multi-target arc field radiation treatment plan, and by one approach, optimization of treatment target projections can be calculated separately for each treatment target and for each different gantry-direction.

By one approach, at least some optimization iterations can include selecting a subset of treatment targets of the plurality of treatment targets that correspond to a selection of control points, wherein the subset of treatment targets represents treatment targets that are to be irradiated in the selection of control points. If desired, multi-leaf collimator leaf positions can be optimized for each treatment target projection in a separate optimization step. By one approach, possible multi-leaf collimator leaf position values can be restricted as a function of default multi-leaf collimator leaf positions.

So configured, these teachings can be especially useful when applied to multi-target arc field treatment plans, where there can be multiple small targets that are treated using multiple arc fields with different angles of incidence. In such an application setting, each arc can be represented as a sequence of control points, and during optimization those sequences can be subdivided into smaller arc sectors for easier optimization.

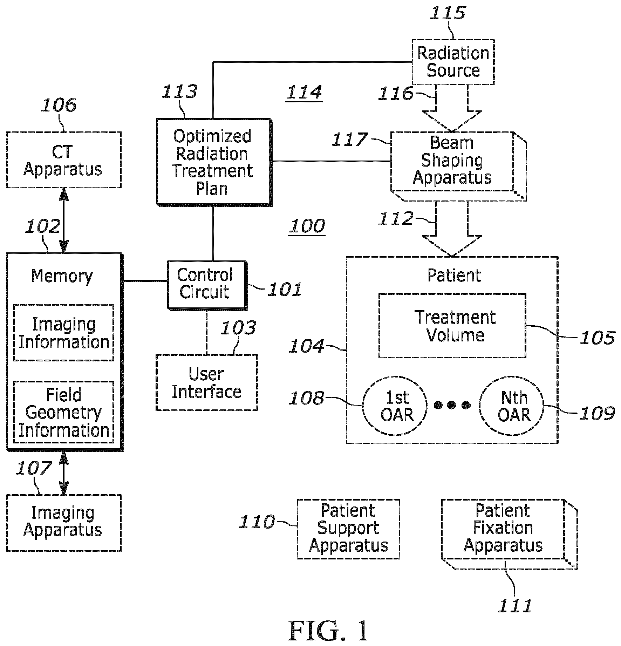

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein. (It will be appreciated that "a" control circuit may physically comprise a plurality of discrete hardware platforms as desired. It will therefore be understood that references to "a control circuit" can refer to either a single hardware platform or a plurality of hardware platforms.)

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

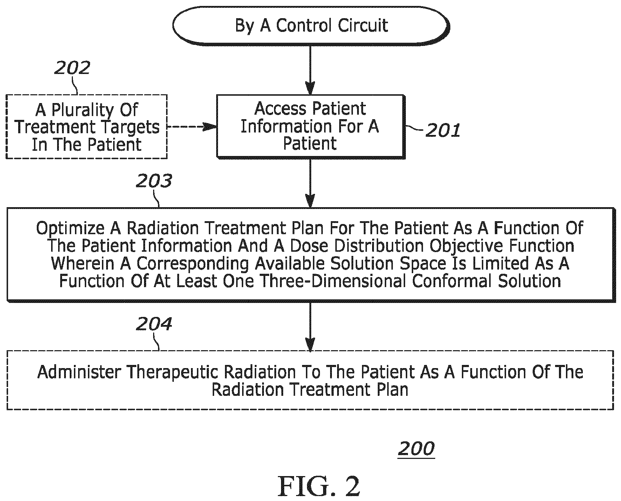

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for the control circuit 101 accessing patient information for a patient (for example, from the aforementioned memory 102). This patient information can comprise, for example, information (including but not limited to patient images) regarding a treatment target and/or one or more organs-at-risk. These teachings will accommodate the patient information including information 202 regarding a plurality of treatment targets in the patient and wherein the radiation treatment plan comprises a multi-target arc field radiation treatment plan. The foregoing includes situations where the patient presents a plurality of relatively small treatment targets.

At block 203, the control circuit 101 then optimizes a radiation treatment plan for the patient as a function of the aforementioned patient information as well as a dose distribution objective function, wherein a corresponding available solution space is limited as a function of at least one three-dimensional conformal solution.

These teachings will accommodate a variety of approaches to so limiting the available solution space. As one example, the corresponding available solution space is limited by, at least in part, limiting the available solution space to a three-dimensional conformal solution. As another example, the corresponding available solution space is limited as a function of at least one three-dimensional conformal solution by, at least in part, limiting the available solution space to three-dimensional conformal partial solutions. And as yet another example, the corresponding available solution space is limited as a function of at least one three-dimensional conformal solution by, at least in part, limiting the available solution space to conformal multi-leaf collimator solutions. It will be understood that these examples are intended to serve an illustrative purpose and are not intended to suggest any particular limitations in these regards.

By one useful approach, the aforementioned optimizing of the radiation treatment plan as a function of the dose distribution objective function can comprise, at least in part, optimizing the radiation treatment plan as a function of inverse treatment planning. In inverse treatment planning, one begins with a desired dose distribution within the patient's body (for example, within a treatment target and/or an organ-at-risk) and one then calculates an optimal beam configuration to achieve that distribution.

As noted above, the patient information may include information regarding a plurality of treatment targets in the patient and wherein the radiation treatment plan comprises a multi-target arc field radiation treatment plan. In such a case, optimization of corresponding treatment target projections can be calculated separately for each treatment target and for each different gantry-direction that correspond to the treatment plan arc.

Also in such a case (and in lieu of the foregoing separate calculations, or in combination therewith), at least some optimization iterations can include selecting a subset of treatment targets of the plurality of treatment targets that correspond to a selection of control points, wherein the subset of treatment targets represents treatment targets that are to be irradiated in the selection of control points. In such a case, multi-leaf collimator leaf positions can be optimized for each treatment target projection in a separate optimization step. (Multi-leaf collimators are known in the art and are comprised of a plurality of individual parts (known as "leaves") that are formed of a high atomic numbered material (such as tungsten) that can move independently in and out of the path of the radiation-therapy beam in order to selectively block (and hence shape) the beam. Typically the leaves of a multi-leaf collimator are organized in pairs that are aligned collinearly with respect to one another and which can selectively move towards and away from one another. A typical multi-leaf collimator has many such pairs of leaves, often upwards of twenty, fifty, or even one hundred such pairs.) If desired, possible multi-leaf collimator leaf position values can be restricted as a function of default multi-leaf collimator leaf positions.

At optional block 204, these teachings will accommodate then administering therapeutic radiation to the patient as a function of the resultant optimized radiation treatment plan using, for example, the aforementioned radiation treatment platform 114.

So configured, these teachings can provide for combining three-dimensional conformal planning, algorithmic treatment strategy optimization, and conventional inverse planning for radiation treatment sequence determination to obtain efficient and robust plans for cases where target dose conformality and short delivery time are more important than a level of plan quality that might be obtained, for example, by using beam modulation via multi-leaf collimator leaves. In these regards, these teachings will accommodate optimizing a radiation treatment plan using inverse treatment planning methods (and hence using, for example, an objective function for the dose distribution) while limiting the available solution space to three-dimensional conformal solutions (or, by one approach, solutions that are at least close to three-dimensional conformal partial solutions where, for example, being "conformal" can mean that the solution in leaf position space of the selected control points consists of leaf positions that remain in the vicinity of the selected target projection boundaries.). (By a possibly different approach, a "close-to-conformal" solution could refer to active leaf positions that are (at least most of the relevant time) in the vicinity of the target projection in all (or at least most) control points (where "active" refers to leaves that are an active part of actually delivering dose as versus, say, closed leaf-pairs for which the closing position is less important in these regards.) That vicinity can refer to active leaves that are not further than some predetermined specified distance such as, say, 3 mm (measured from the Beam-eye-view projection to isocenter distance) from some part of the target projection, or within some predetermined percentage distance (such as, for example, 0% ,10%, or 20%) of the width of the target projection.

In the foregoing regards, conformality can refer, if desired, to adjacent leaves that act as a smooth boundary and that are not individually modulating back and forth. One possible metric to be applied in the foregoing regards is the surface area of the projection that is being covered by the leaves at any given control point. These teachings would accommodate, in such a case, specifying that one metric for "close to" is that the leaf opening at the control point reveals at least 70%, 80%, 90%, or 100% (or any particular percentage within that range) of the selected target projection.

Further details that comport with these teachings will now be presented. It will be understood that the specific details of these examples are intended to serve an illustrative purpose and are not intended to suggest any particular limitations with respect to these teachings.

By one approach, these teachings can be applied in a Stereotactic Radio Surgery (SRS) planning method where the objective function typically benefits from high dose gradients (i.e., dose falloff) outside the target boundary and where the solution space is limited to conformal multi-leaf collimator apertures (or, if desired, apertures that are nearly conformal). In this context, a "conformal aperture" means an aperture that is conformal to the target projection on the multi-leaf collimator plane. By one approach, and in the case of multiple targets, the target projection can be a projection of a subset of targets.

By one approach, these teachings can be viewed as comprising an inverse planning optimization (such as volumetric modulated arc therapy (VMAT) as a user-defined cost function can be minimized by iteratively searching possible leaf-sequences (and associated meter set weights). In addition, however, these teachings can provide for a different way of searching the leaf-position space. Some illustrative examples in those regards will now be presented.

During an initial phase of the optimization process the target projections are calculated separately for each target and for each different gantry-direction. By one approach, default multi-leaf collimator leaf positions can be determined for each target separately. (If desired, in a general case these target projections can be allowed to overlap.)

The optimization iteration process can include a step where a subset of targets is selected for a selection of control points. In this example, this subset represents the target or targets that are going to be irradiated in those control points. Various strategies can be used for how the set of control points is selected. By one approach, one iteration can focus only on a subset of control points. By another approach, the subset of targets can be specified to be same for a set of a certain amount of consecutive control points. By one approach, specified constraints can be followed for each subset of targets. For example, it can be specified that the subset needs to be selected so that any two of the targets in the subset do not share a leaf-pair that would be needed for conformal treatment for both targets. If desired, this step can also include consideration of other discrete optimization parameters, such as, but not limited to, beam energy.

If desired, the foregoing step can include, when selecting the subset of targets and in addition to the cost function evaluation, also utilizing geometrical rules to restrict the valid subsets. Examples of other possible geometrical rules include, but are not limited to:
Avoiding selecting target pairs that are too close to each other (using a threshold distance of choice) to facilitate more aggressive avoidance of so-called dose bridging;
Avoiding selecting targets that are too deep within the patient (i.e., where the rays must travel more than a predetermined threshold distance through normal tissue before reaching the target);
Taking into account the time it takes to move leaves from one target to another, essentially enforcing that if two target apertures do share a leaf-pair, there needs to be at least a certain time period between the closing of the first target aperture and the opening of the second target aperture; and/or
Establishing a bias to provide larger angular separation of incoming rays for each target.

In another optimization step, the multi-leaf collimator leaf positions can be optimized for each target projection separately. In this step, the possible leaf position values can be restricted to the vicinity of the default multi-leaf collimator leaf positions (for example, a specified deviation from the default position can be allowed). Examples of possibly useful "vicinity" thresholds are any value between, say, 0.5 mm to 5.0 mm. In lieu of the foregoing, or in combination therewith, a relative measure X % of the target projection width at a particular control point could be employed, such as, for example, 0%, 10%, or 20% (or any value within that range). Also, to be conformal (for example, by keeping the adjacent leaves close to each other to yield a smooth aperture), such a relative measure could serve as a constraint for a group of adjacent leaves. As an example, if a selection of leaves is allowed to find a solution in the vicinity of the default positions, those solutions could be limited to leaf positions that allow the same relative movement from their individual default positions. In other words, the leaves would need to move as a group. By one approach, this step can also accommodate parameters that are common for all leaves in the control point, such as the meter set weight, jaw positions, or collimator angle.

If desired, and referring to the aforementioned subset-selection step and the separate optimization step of multi-leaf collimator leaf positions for each target projection separately, these teachings will accommodate not necessarily performing each with equal cadency. These teachings will accommodate, for example, that for every subset-selection step the leaf position optimization step runs multiple times (or, by another approach, not at all in some iterations).

Referring still to the aforementioned subset-selection step and the separate optimization step of multi-leaf collimator leaf positions for each target projection separately, by one approach these two activities can utilize different optimization strategies. The former step can benefit from a relatively small set of possible choices of the discrete optimization parameters while the latter step could use, for example, a gradient-based optimization method where the cost function gradient is projected through dose space and fluence space to a request to move leaves inwards or outwards. In this application setting, the limited position of acceptable leaf positions can facilitate the projection of the gradient to the leaf-position space.

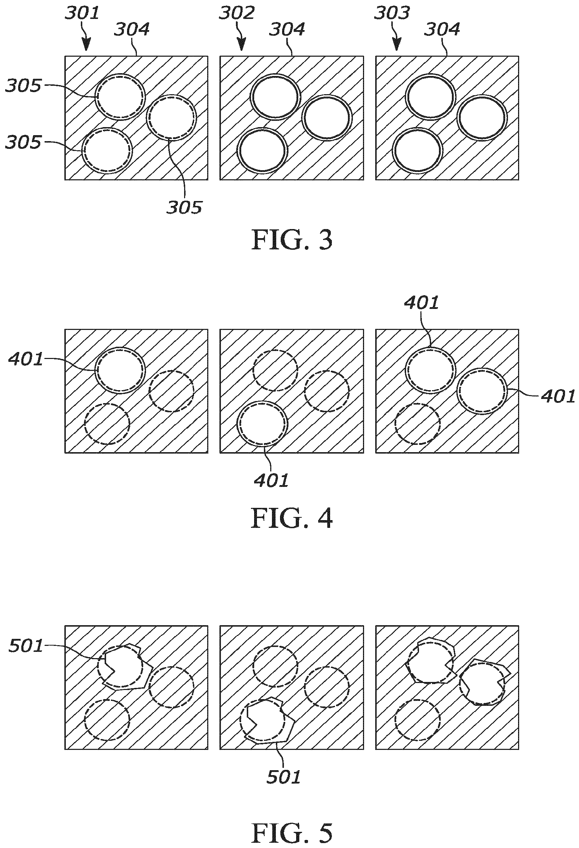

FIG. 3 presents a schematic presentation of the course of optimization when observed from the Beam-eye-view of a certain control point (gantry angle). The columns 301-303 correspond to different potential paths that the optimizer can take. The boxes 304 represent the multi-leaf collimator plane in a given corresponding control point, in which the leaves move in the direction of the stripes (in this illustrative example, that direction being a 45 degree angle). The dashed-line circles 305 represent three target structure projections, and the actual multi-leaf collimator aperture (i.e., the leaf openings) are represented as solid lines.

FIG. 3 presents, in particular, initialization of the optimizer before a first actual iteration. In this example, the multi-leaf collimator apertures (depicted as the solid-line circles) at this stage are defined just for book-keeping, which means that there is no issue should some leaves be active in more than one target projection outline.

FIG. 4 depicts the target selection phase of the optimization iteration, where only a sub-set of all target projections is taken. Pursuant to these teachings, geometrical constraints (in this illustrative example, that one leaf-pair can contribute to only one target projection) restrict the possible choices in each instance. The selected subset(s) in each of the possible paths is denoted here by reference numeral 401.

FIG. 5 illustrates that the leaf-aperture optimization described herein allows for deviations from the purely conformal leaf positions (as illustrated in some cases by reference numeral 501).

It will be appreciated that these teachings permit a combination of the benefits of a three-dimensional conformal approach (such as low Monitor Units in addition to fast delivery and an intuitively understandable relation between field geometry and dose distribution) with some of the benefits of inverse planning (such as an ability to state desired qualities of the planning via the cost function as well as achieving automatically optimal leaf-positions). It will also be appreciated that these teachings are particularly suitable for multi-target SRS (or stereotactic body radiation therapy (SBRT)) treatments where target dose homogeneity often does not play a crucial role (and therefore does not require excessive leaf-modulation deep inside the target aperture).

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method comprising:
by a control circuit:
accessing patient information for a patient;
optimizing a radiation treatment plan for the patient as a function of the patient information and a dose distribution objective function wherein a corresponding available solution space is limited as a function of at least one three-dimensional conformal solution.

2. The method of claim 1 wherein the corresponding available solution space is limited as a function of at least one three-dimensional conformal solution by, at least in part, limiting the available solution space to a three-dimensional conformal solution.

3. The method of claim 1 wherein the corresponding available solution space is limited as a function of at least one three-dimensional conformal solution by, at least in part, limiting the available solution space to three-dimensional conformal partial solutions.

4. The method of claim 1 wherein the corresponding available solution space is limited as a function of at least one three-dimensional conformal solution by, at least in part, limiting the available solution space to conformal multi-leaf collimator solutions.

5. The method of any one of claims 1 to 4 wherein optimizing the radiation treatment plan as a function of the dose distribution objective function comprises, at least in part, optimizing the radiation treatment plan as a function of inverse treatment planning.

6. The method of any one of claims 1 to 5 wherein the patient information includes information regarding a plurality of treatment targets in the patient and wherein the radiation treatment plan comprises a multi-target arc field radiation treatment plan.

7. The method of claim 6 wherein optimization of treatment target projections are calculated separately for each treatment target and for each different gantry-direction.

8. The method of claim 6 or 7 wherein at least some optimization iterations include selecting a subset of treatment targets of the plurality of treatment targets that correspond to a selection of control points, wherein the subset of treatment targets represents treatment targets that are to be irradiated in the selection of control points.

9. The method of claim 8 wherein multi-leaf collimator leaf positions are optimized for each treatment target projection in a separate optimization step.

10. The method of claim 9 wherein possible multi-leaf collimator leaf position values are restricted as a function of default multi-leaf collimator leaf positions.

11. An apparatus comprising:
a control circuit configured to:
access patient information for a patient; and
optimize a radiation treatment plan for the patient as a function of the patient information and a dose distribution objective function wherein a corresponding available solution space is limited as a function of at least one three-dimensional conformal solution.

12. The apparatus of claim 11 wherein the control circuit is configured to limit the corresponding available solution space as a function of at least one three-dimensional conformal solution by, at least in part, limiting the available solution space to a three-dimensional conformal solution.

13. The apparatus of claim 11 wherein the control circuit is configured to limit the corresponding available solution space as a function of at least one three-dimensional conformal solution by, at least in part, limiting the available solution space to three-dimensional conformal partial solutions.

14. The apparatus of claim 11 wherein the control circuit is configured to limit the corresponding available solution space as a function of at least one three-dimensional conformal solution by, at least in part, limiting the available solution space to conformal multi-leaf collimator solutions.

15. The apparatus of any one of claims 10 to 14 wherein the control circuit is configured to perform the method of any one of claims 5 to 10.
